# EUROPEAN PATENT SPECIFICATION

(11) **EP 1 836 951 B1**
(45) Date of publication and mention of the grant of the patent: **14.10.2009**
(21) Application number: 07012487.0
(22) Date of filing: 24.05.2006
(51) Int. Cl.: A61B 1/12, A61B 1/015, A61M 1/00, A61M 3/02

(54) **Water feeding device for an endoscope**
Wasserversorgungsvorrichtung für ein Endoskop
Dispositif d'alimentation en eau pour endoscope

(30) Priority: 25.05.2005 JP 2005152023; 25.05.2005 JP 2005152024; 25.05.2005 JP 2005152025
(43) Date of publication of application: 26.09.2007
(62) Divisional of application: 06010715.8
(73) Proprietor: Fujinon Corporation, Saitama-shi, Saitama (JP)
(72) Inventor: Akiba, Haruo C/O fUJINON cORPORATION, Saitama-shi, Saitama (JP); Machiya, Mamoru c/o Fujinon Corporation, saitama-shi, saitama (JP)
(74) Representative: Klunker . Schmitt-Nilson . Hirsch

(56) References cited:
- US-A- 4 940 457
- US-A- 5 273 524
- US-A- 5 836 909

## Description

### Background of the Invention

### 1. Field of the Invention

The present invention relates to a water feeding device for an endoscope, and particularly to a water feeding device for an endoscope for supplying liquid into an object under observation by using a dedicated water feeding pipe line disposed in the endoscope and any kind of water feeding pipe line inserted through a treatment instrument insertion channel of the endoscope provided to the treatment instrument inserted through the treatment insertion channel of the endoscope.

### 2. Description of the Related Art

In the related-art endoscope device, a dedicated water feeding pipe line (so-called water jet pipe) is provided in an endoscope (scope), and cleaning physiological saline or drug solution for medical treatments is supplied through such water feeding pipe line to an object under observation (diseased part or the like) by a water feeding pump portion. The water feeding pipe line is disposed separately from an air-feeding/water-feeding pipe line for cleaning an observation window of an object optical system.

The endoscope device is provided with a treatment instrument insertion channel through which various kinds of treatment instruments can be introduced into the object under observation. Also, liquid corresponding to the condition of an object under observation may be is injected directly through the treatment instrument insertion channel, or a water feeding tube is inserted into the treatment instrument insertion channel and then the liquid is injected through the water feeding tube. In that type of endoscope device, a water feeding tube having a different diameter from that of the water jet pipe is used, and thus there is an advantage that the water feeding range (the breadth of the water feeding port), the water feeding amount or the water feeding speed can be selected in connection with the situation.

A treatment instrument having a water feeding pipe line has been recently used. For example, with respect to a high-frequency knife for ablating a living tissue by high-frequency incision, cleaning water such as physiological saline or the like is fed from an affixed water feeding pipe line during cautery incision to clean a blood-issued part or the like.

Further, from US patent 4,940,457 an irrigation system for use during arthroscopy is known having a control unit with a pump for supplying fluid from reservoir bags to the arthroscopic device, wherein the pump is controlled by pressure transducer which gives feedback on the fluid pressure via a return tube.

### Summary of the Invention

When there is used a dedicated in-endoscope water feeding (water jet) pipe line or a water feeding pipe used while inserted through the treatment instrument channel is used, dust or materials or the like of the object under observation clogs at the injection port of the water feeding pipe or a synthetic resin tube between the endoscope and the water feeding pump portion may be trodden in some cases. In such a case, the water feeding pressure in the pipe increases abnormally, and thus there occurs a disadvantage that the various kinds of water feeding pipes containing the water jet pipe are damaged or the water feeding tube is detached at the connection portion.

Recently, not only the water jet pipe, but also various kinds of water feeding pipes using the treatment instrument insertion channels have been used, and thus the constructions of the pipe lines have been complicated. Furthermore, the water feeding conditions of the flow rate, the pressure, etc. have been complicated, and thus the probability that the above disadvantage occurs has been higher and higher.

The present invention has been implemented in view of the foregoing problem, and has an object to provide a water feeding device for an endoscope in which the water feeding pressure in a pipe can be prevented from increasing abnormally, and damage of water feeding pipes such as a water jet pipe, etc. and detachment of a water feeding tube at a connection portion can be prevented.

In order to attain the above object, according to a first aspect of the invention, a water feeding device for an endoscope, comprises: a water feeding pump portion that supplies liquid in a storage portion by a pump; a water feeding pipe line that is disposed so as to pass from the water feeding pump portion through the endoscope and supplies liquid to an object under observation; and one of an orifice and an automatic opening/closing type pressure relief valve, that limits the maximum water feeding pressure (the pressure of fluid in a pipe line) and is provided to a water feeding pipe line disposed between the water feeding pump portion and the endoscope.

The water feeding device for the endoscope further comprises a pipe line that returns liquid flowing out from said one of the orifice and the automatic opening/closing type pressure relief valve to the storage portion, the pipe line being provided between: one of the orifice and the pressure relief valve; and the storage portion.

According to the construction of the first aspect of the invention, the liquid flowing out from the orifice or pressure relief valve is returned to the storage portion by the return pipe line (feedback path), and thus the liquid is re-used.

According to a second aspect of the invention, in the first aspect of the invention, at least one of a first water feeding pipe line connected to the water feeding pipe line in the endoscope and a second water feeding pipe line to be introduced through a treatment instrument insertion channel of the endoscope is connected to the water feeding pump portion, and said one of the orifice and the automatic opening/closing type pressure relief valve is provided to an output port pipe connected to said at least one of the first and second water feeding pipe.

According to the first and second aspects of the present invention, when the pressure in the water feeding pipe line is equal to or more than the maximum water feeding pressure set by the orifice or pressure relief valve, the liquid flows out from the orifice or pressure relief valve, so that the pressure equal to or higher than the maximum water feeding pressure can be prevented from being applied to the water feeding pipe line.

According to the first and second aspect of the water feeding device for the endoscope of the present invention, since the water feeding pressure does not abnormally increase, the water jet pipe or each kind of water feeding pipe used while inserted through the treatment instrument insertion channel is neither damaged nor broken, and the water feeding tube is prevented from being detached at the connection portion, so that excellent use of the observation and the treatment can be secured.

### Brief Description of the Drawings

Fig. 1 is a diagram showing the construction when the water feeding device for an endoscope according to the example 1-1 which is not part of the present invention is applied;
Figs. 2A and 2B is a diagram showing the change-over operation of a change-over valve according to 1-1;
Fig. 3 is a diagram showing the construction when a water feeding device for an endoscope according to the example 1-2 which is not part of the present invention is applied to an endoscope;
Fig. 4 is a diagram showing the detailed construction of the water feeding device for the endoscope according to 1-2;
Fig. 5 is a perspective view showing the construction of a water feeding pump portion and a tank according to the device of 1-1;
Fig. 6 is a diagram showing the construction when the water feeding pup portion and the tank of 1-1 are arranged in a system rack;
Fig. 7 is a diagram showing the relationship between the operation of a foot switch [(A)] and a water feeding amount [(B)] of a water jet for the water feeding;
Fig. 8 is a partially cross-sectional view showing the construction of the tank;
Fig. 9 is a diagram showing the construction of a water feeding device for an endoscope according to the embodiment called embodiment 2 hereinafter 2 of the present invention;
Fig. 10A shows a crass-sectional view of an orifice of the embodiment 2;
Fig. 10B shows a diagram when viewed from the inside of the pipe line on the orifice of the embodiment 2;
Fig. 11 is a diagram showing the construction of a pump and the construction of a circuit for flow rate adjusting control in the embodiment 2;
Fig. 12 is a diagram showing the construction of a pressure relief valve arranged in the device of the embodiment 2;
Fig. 13 is a diagram showing the construction of a water feeding device for an endoscope according to the example 3;
Fig. 14 is a diagram showing another construction of the liquid container; and
Fig. 15 is a diagram showing the construction of the related-art liquid tank.

### Detailed Description of the Invention

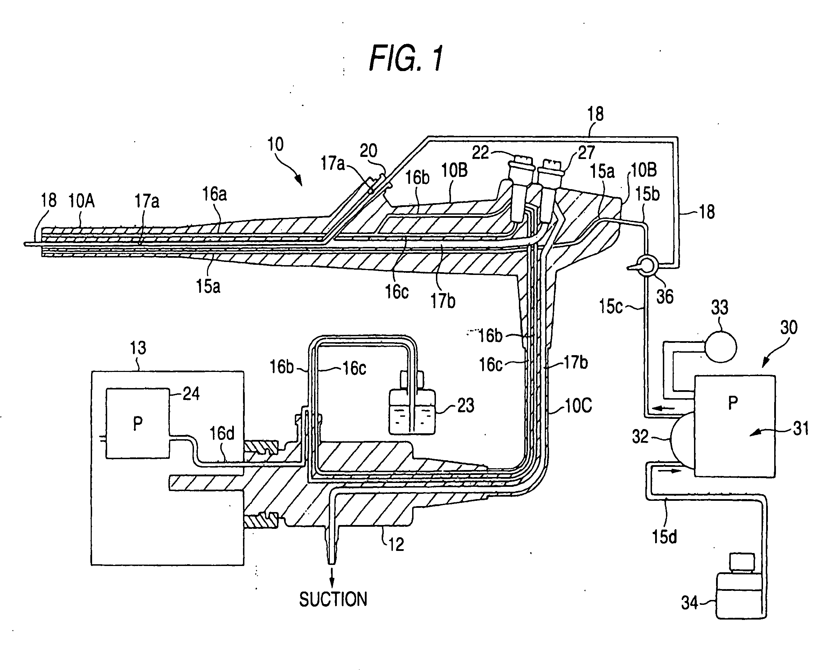

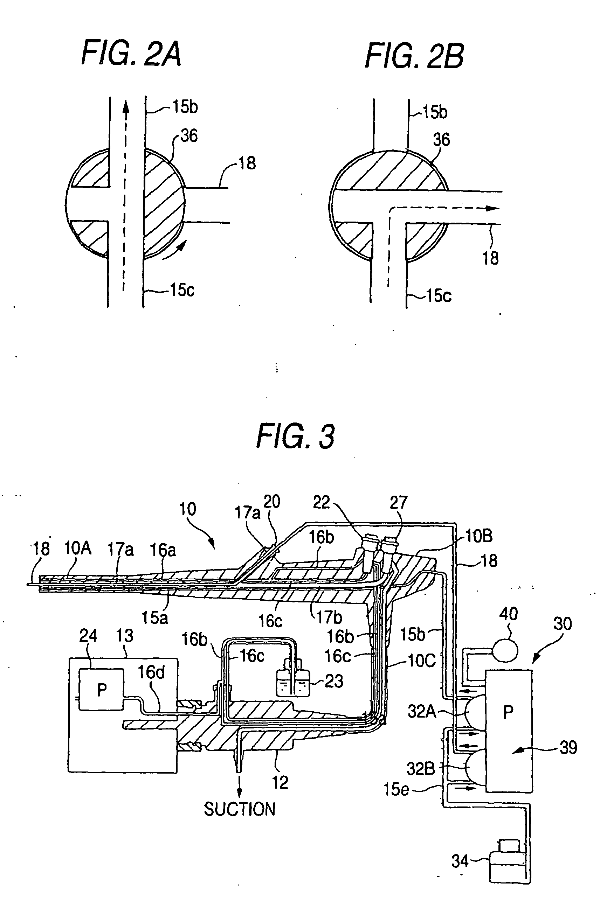

Figs. 1 and 2 show the construction of a water feeding device for an endoscope according to 1-1. As shown in Fig. 1, an endoscope (electronic endoscope) 10 has an insertion portion 10A, an operating portion 10B and a cable portion 10C, and it is connected to a light source device 13 shown in Fig. 1 by a connector 12 of the cable 10C. In the case of the electronic endoscope, the cable 10C is also connected to a processor device. Various kinds of pipe lines are provided in the endoscope 10. A water jet pipe (dedicated water feeding pipe line) 15a is disposed so as to extend from the tip of the insertion portion 10A to the rear end portion of the operating portion 10B. An air-feeding/water-feeding pipe 16a is provided at the tip side to clean an observation window (lens face) of an object optical system equipped to the tip of the insertion portion 10A through a nozzle, and an air-feeding pipe 16b and a water feeing pipe 16c are provided so as to intercommunicate with the air-feeding/water-feeding pipe 16a.

The endoscope 10 is equipped with a treatment instrument insertion channel 17a which also serves as a suction pipe, and a suction pipe 17b intercommunicating with the treatment instrument insertion channel 17a, and the treatment instrument insertion channel 17a is provided with a forceps port (treatment instrument insertion port) 20 is provided at the position corresponding to the operating portion 10B. A second water feeding pipe 18 is inserted from the forceps port 20 into the treatment instrument channel 17a, and water feeding tubes formed of hard synthetic resin and having various diameters and water feeding pipes provided for treatment instruments such as a high-frequency knife, etc. exist as the second water feeding pipe 18.

An air-feeding/water-feeding operation valve (switch) 22 is provided at some midpoint in the air-feeding pipe 16b and the water-feeding pipe 16c for cleaning the observation window describedabove, andatank (firstwaterfeedingtank) 23 is connected to the air-feeding pipe 16b and the water-feeding pipe 16c. The air-feeding pipe 16b is connected through the connection pipe 16d to a pump 24 inside (separately from) the light source 13, and air feeding and water feeding to the observation window are carried out by a two-step pushing operation of the air-feeding/water-feeding operation valve 22, for example. Furthermore, the suction pipe 17b is provided with a suction operation valve (switch) 27 at some midpoint thereof, and it is connected to a suction device through the connection pipe of the connector 12. By operating the suction operation valve 27, contents in the object under observation can be sucked (through 17a, 17b) and discharged.

The water feeding device for the endoscope is equipped with a water feeding pump portion 30 for feeding liquid into the object under observation, and the water feeding pump portion 30 is quipped with a pump portion main body 31 having a motor and a control circuit, a pump 32 disposed at the front side of the main body 31, a foot switch 33 for carrying out a water feeding operation and a tank (second water feeding tank) 34. The tank 34 is connected to the pump 32 (input port) through the connection pipe 15d. The pump 32 is designed so that a soft water feeding tube is disposed circumferentially on outer periphery of a rotor, a pressing member which rotationally moves while crushing the tube is secured on the outer periphery of the rotor, and liquid in the tube is pushed out by rotating the rotor, thereby feeding water.

In this example, the first water feeding pipe 15b and the connection pipe 15c are provided to connect the pump 32 (the output port) and the water jet pipe 15a disposed in the endoscope 10, and the pipe line change-over valve 36 (1 input and 2 outputs) comprising the three-way turncock, a choke valve, etc. is provided between the first water feeding pipe 15b and the connection pipe 15c. The connection pipe 15c at the pump 32 side is connected to the one input of the change-over valve 36, and the first water feeding pipe 15b and the second water feeding pipe 18 are connected to the two outputs of the change-over valve 36. That is, the water feeding operation using the water jet pipe 15a in the endoscope and the water feeding operation using the second water feeding pipe 18 corresponding to each water feeding tube having each diameter or a water feeding pipe provided to a treatment instrument such as a high-frequency knife or the like, which is introduced from a forceps port 20 through a treatment instrument insertion channel 17a into an object under observation are selectively switched to eachotherandusedbymanuallyoperating the change-over valve 36.

Fig. 2 shows the operation state of the change-over valve 36, wherein Fig. 2(A) shows a state that the connection pipe 15c at the pump 32 side and the first water feeding pipe 15b are connected to each other (the pipes are opened) by operating the lever of the change-over valve 36 (the secondwater feedingpipe 18 is closed), and when the lever is counterclockwise turned from this state by 90 degrees, the connection pipe 15c at the pump 32 side and the second water feeding pipe 18 are connected to each other (the first water feeding pipe 15b is closed).

According to 1-1 as described above, any one of the water feeding operation using the water jet pipe 15a (first water feeding pipe 15b) provided in the endoscope and the water feeding operation using the second water feeding pipe 18 comprising each water feeding tube having each diameter or the water feeding pipe provided to the treatment instrument such as the high-frequency knife or the like which is introduced from the forceps port 20 through the treatment instrument insertion channel 16a into the object under observation can be selected by manually operating the mechanical change-over valve 36, and the water feeding operation under various conditions can be executed in accordance with the observation/treatment condition. The water feeding operation can be carried out on both the pipes 15a and 18 by operating the change-over valve 36.

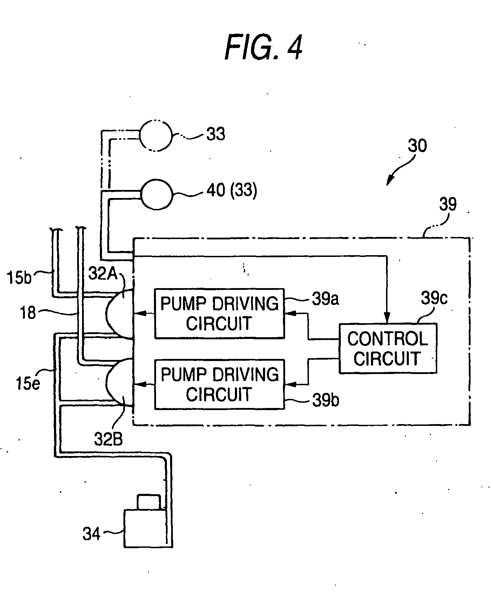

Figs. 3 and 4 shows the construction of a water feeding device for an endoscope according to 1-2. In this second example, a pump for a first water feeding pipe line and a second water feeding pipeline are provided. As shown in Figs. 3 and 4, the pump portion main body 39 is equipped with a first pump 32A and a second pump 32B. The first water feeding pipe 15b is connected to the output port of the first pump 32A, and the second water feeding pipe 18 is connected to the output port of the second pump 32B. A tank 34 is connected to the input ports of the first and second pumps 32A, 32B by a connection pipe 15e.

Furthermore, as shown in Fig. 4, pump driving circuits 39a, 39b containing motors for driving the first and second pups 32A, 32B, and a control circuit 39c for receiving a signal from a foot switch 40 serving as a water feeding operation switch and controlling the switching operation between the two pump driving circuits 39a and 39b are provided in the pump portion main body 39. The foot switch 40 comprises a two-step push switch. The control circuit 39c drives the first pump 32A by one-step push, and also drives the second pump 32B by two-step push. Two foot switches 33 having the same construction as 1-1 may be arranged as the water feeding operating switch in connection with the two pumps 32A, 32B

According to 1-2 as described above, one of the first pump 32A and the second pump 32B is operated by operating the foot switch 40, and any one of the water feeding operation using the water jet pipe 15a (and the first water deeding pipe 15b) and the water feeding operation using the second water feeding pipe 18 which is inserted through the treatment instrument insertion channel 17a and corresponds to the water feeding tube of each diameter or the water feeding pipe provided to the treatment instrument such as a high-frequency knife or the like can be selected, and the water feeding under each condition corresponding to the observation/treatment condition can be executed. Both the pumps 32A and 32B can be operated.

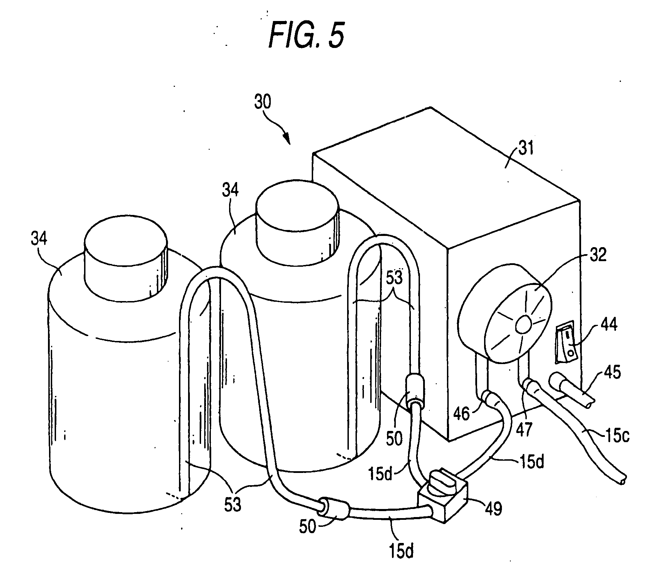

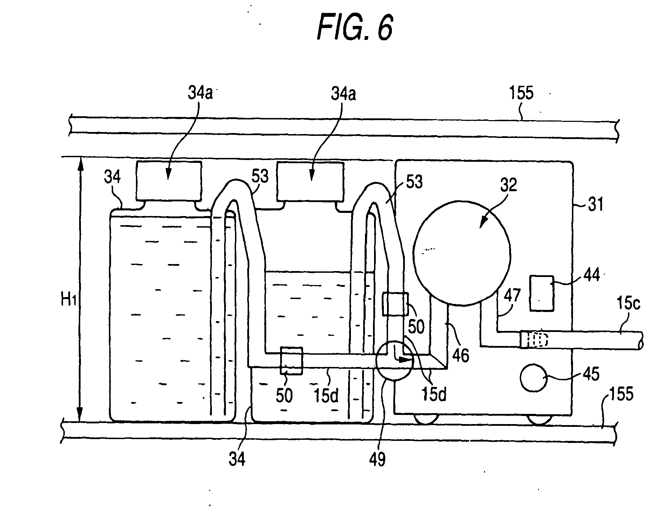

Figs. 5 to 8 show the specific construction of the water feeding pump portion and the tank in 1-1 (which is also applicable to 1-2). As shown in Figs. 5 and 6, the main body 31 of the water feeding pump portion (device) 30 is equipped with a power supply switch 44, a connecting portion 45 of the foot switch 33, etc. Furthermore, the connection pipe 15d at the tank side is connected to the input port 46 of the pump 32, and the connection pipe 15c at the endoscope side is connected to the output port 47 of the pump 32. In this example, the connection pipe 15c (water feeding output side) and the first water feeding pipe 15b connected to the water jet pipe 15a and the first water feeding pipe 15b connected to the water jet pipe 15a are formed of hard synthetic resin pipes, for examples, pipes formed of materials such as Teflon (registered trademark), polytetrafluoroethylene (fluorocarbon resin) or the like, which are never deformed or little deformed even when the water feeding pressure is high. The second water feeding pipe 18 is also likewise formed of a synthetic resin pipe.

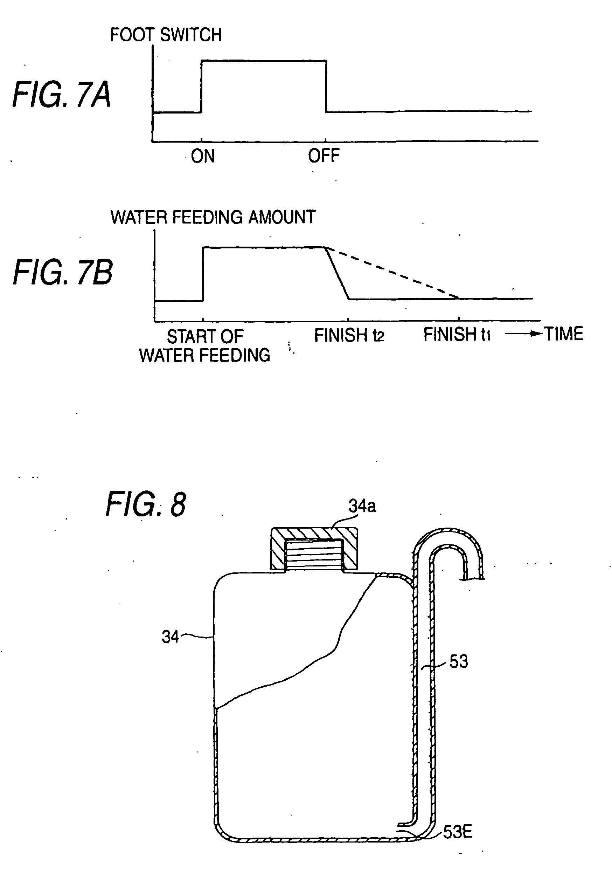

According to the connection pipe 15c and the first water feeding pipe 15b which are formed of such hard synthetic resin pipes, after the water feeding operation is stopped by the foot switch 33, the residual water in the pipes is prevented from flowing out from the outlet of the water jet pipe 15a at the tip of the endoscope. Fig. 7 shows the relationship between the operation of the foot switch 33 and the water feeding amount from the outlet of the water j et pipe 15a. In the related-art endoscope in which a soft synthetic resin pipe such as a silicon tube or the like is used for the connection pipe (15c), even when the foot switch 33 is set to off [(A) of Fig. 7], the expanded soft synthetic resin pipe is contracted, so that the water feeding of the residual water in the pipe continues until a finish time t₁ (from several seconds to eight seconds, for example) as indicated by a dotted line of (B) of Fig. 7. However, according to the hard synthetic resin connection pipe 15c of this example, even after the foot switch 33 is set to off, the water feeding of the residual water is immediately finished at a finish time t₂ (within one second) as indicated by a solid line of (B) of Fig. 7, so that the creep phenomenon can be greatly improved.

In Figs. 5 and 6, the connection pipe 15d connected to the input port 46 of the pump 32 is designed to be bifurcated through a pipe line change-over valve (three-way turncock or the like, 2 inputs, 1 output) 49. (Liquid) take-out pipes 53 of two tanks 34 are connected to the bifurcated connection pipe 15d through Luer-locks 50 serving as rotational spiral coupling type connectors. Accordingly, when all the liquid in one of the tanks 34 is consumed, the liquid in the other tank 34 may be used by switching the input pipe line of the change-over valve 49. During use of one of the tanks 34, the other tank 34 may be supplemented with liquid or exchanged by a new tank 34.

Furthermore, the tank 34 is formed so as to have a capacity of 1000ml or more. That is, the tank capacity of the prior art is set to about 500ml. Since liquid of 200 to 300ml or more is used for each case to clean mucous membrane, etc. of a site under observation in the observation and treatment using the endoscope, and thus there has frequently occurred such a case that the tank does not satisfy the liquid amount necessary for three cases. However, this example has a capacity of 1000ml or more, and thus the liquid amount required for four cases or more canbe secured.

Furthermore, as shown in Fig. 6, the tank 34 is designed so that the height thereof is set to be substantially equal to or less than the height H₁ of the pump portion main body (housing) 31, whereby the arrangement of the parts mounted in a system rack or the like can be efficiently performed. That is, in the endoscope device, not only the endoscope (scope), but also various devices such as a processor device, a light source device, a recording device, a pump for feeding air and water to the observation window, a tank, etc. are provided, and it is required to mount these devices in a system rack having a multiple-step accommodating space or the like. Therefore, the height of the tank 34 is set to be substantially equal to (or lower than) the pump portion main body 31 as shown in Fig. 6, whereby these parts are efficiently accommodated in the accommodating space surrounded by the partition plate 155 of the system rack or the like.

Fig. 8 shows the construction of a liquid take-out amount (outlet pipe) of the tank 34. According to the tank 34, the liquid take-out pipe is not formed so as to extend from the cap 34a as shown like the prior art (as shown in the tank 23 of Fig. 1), but it is formed along the side surface of the tank so that a part of the take-out pipe 53 is formed integrally from the upper portion to the bottom surface of the tank, and a pipe port 53E is provided to the bottom surface portion of the tank. According to this construction, the take-out pipe- 53 does not affect the height of the tank 34. Accordingly, as compared with a case where the take-out pipe 53 is disposed in the cap 34a, the position of the upper end of the cap 34a can be set to the height H₁ of the pump portion main body 31, and thus the tank capacity when the height of the tank 34 is set to be substantially equal to the height of the pump portion main body 31 can be achieved, so that this example is effective to even a case where the capacity is set to 1000ml or more.

### [Embodiment 2]

The overall construction of the water feeding device for an endoscope according to the embodiment 2 is same as in the embodiment 1 (See Fig. 1).

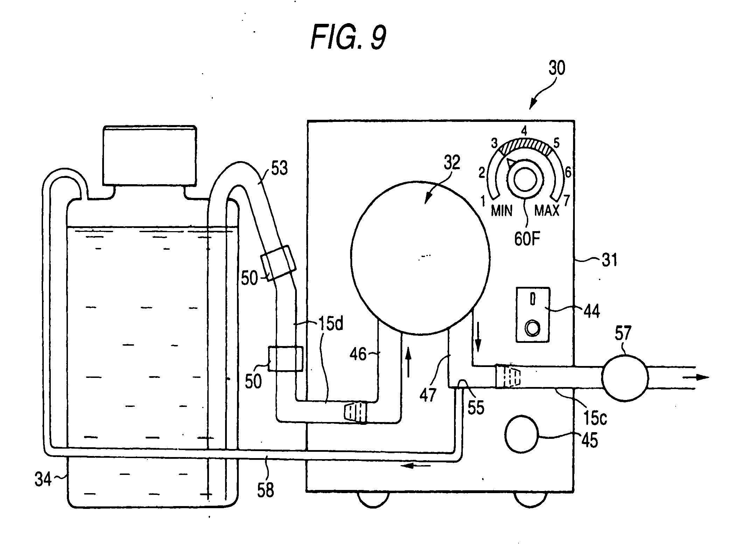

Fig. 9 shows the detailed construction of the water feeding pump portion 30 and the tank 34 on the embodiment 2. As shown in Fig. 1, a power supply switch 44, a connection portion 45 of a foot switch 33, etc. are provided in the main body 31 of the water feeding pump portion (device) 30. In addition, the connection pipe 15d at the tank side is connected to the input port pipe 46 of the pump 32, and a liquid take-out pipe 53 of the tank 34 is connected to the connection pipe 15d through a Luer lock 50. The connection pipe 15c to be connected to the endoscope side is connected to the output port pipe 47 of the pump 32.

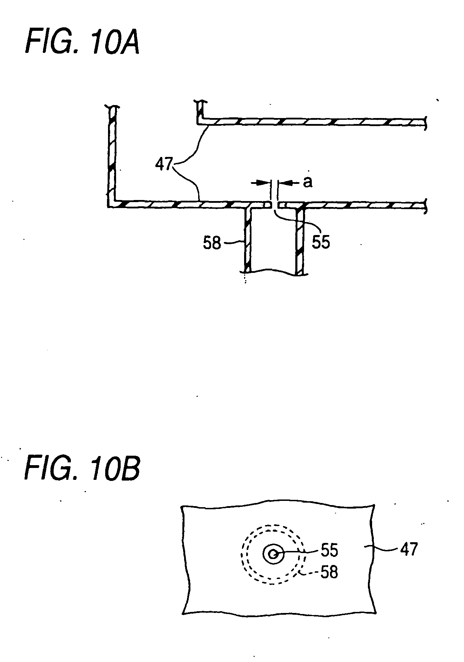

In the embodiment 2, an orifice 55 for setting the maximum water feeding pressure in the pipe is provided to the output port pipe 47 of the pump 32. Figs. 10A and 10B show the construction of the orifice 55. The output port pipe 47 is formed of a hard synthetic resin pipe, and the orifice (hole, opening) 55 comprising a hole of a mm in diameter is formed in the side surface of the synthetic resin pipe. The diameter a of the orifice 55 is set in consideration of the hardness of the synthetic resin pipe of the output port 47 so that prescribed pressure of 29.4 Pascal or less is the maximum water feeding pressure. According to this orifice 55, when the pressure in the water feeding pipe (15a to 15c, 18) is equal to the predetermined maximum water feeding pressure or more, the liquid in the pipe is made to flow out to the outside, whereby the in-pipe pressure can be kept to be less than the maximum water feeding pressure. The orifice 55 may be formed, not in the output port pipe 47, but at some midpoint of the connection pipe 15c, the first water feeding pipe 15b or the second water feeding pipe 18 or the connection portion thereof or the like.

According to the embodiment 2, as shown in Fig. 9, a check valve 57 is provided at some midpoint of the connection pipe 15c, and also a return pipe 58 for returning fluid to the tank 34 is secured between the orifice 55 and the tank 34. That is, when the pressure in the water feeding pipe becomes the maximum water feeding pressure or more, the liquid which flows out from the orifice 55 between the check valve 57 and the pump side is returned to the tank 34 and re-used.

An orifice having a predetermined diameter may be formed as the orifice 55 by crushing a soft synthetic resin tube with a pinch valve or the like. In this case, the tube itself constituting the orifice is set as the return pipe 58, -or the tube is connected to the return pipe 58.

According to the construction of the embodiment 2, the water jet pipe 15a and the second water feeding pipe 18 inserted from the forceps port 20 through the treatment instrument insertion channel 17a are switched to each other by the change-over valve 36, and target liquid can be fed into the object under observation through the selected water feeding pipe. At this time, when the in-pipe pressure of the water feeding pipe (15a to 15c, 18) is equal to the maximum water feeding pressure or more, the liquid is made to flow out to the external by the orifice 55, whereby the in-pipe pressure can be kept to be less than the maximum water feeding pressure. Furthermore, the liquid flowing out from the orifice 55 is returned into the tank 34, and re-used. At this time, the back flow of the liquid from the water feeding pipes 15a to 15c, 18 is suppressed by the check valve 57. For example, liquid which is not kept under a good sanitary conditions is prevented from being returned from the endoscope side to the tank 34 and re-used.

In the embodiment 2, as shown in Fig. 9, a flow rate adjusting finger grip (a variable-resistance finger grip) 60F is provided to the front surface of the pump portion main body 31. An arcuate graduated index is provided around of the flow rate adjusting finger grip 60F, and the flow rate of water to be fed can be steplessly adjusted by the variable resistor (60).

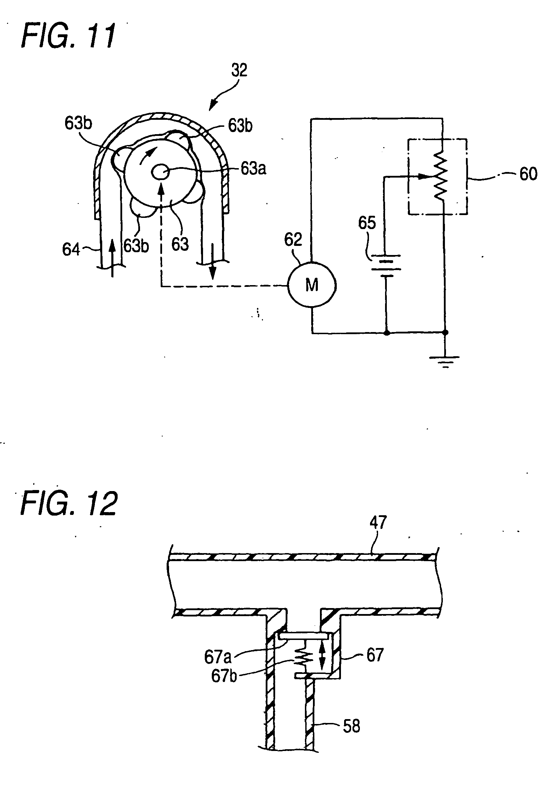

Fig. 11 shows the construction of the pump 32 and the construction of a circuit for flow rate adjusting control. As shown in Fig. 4, in the pump 32, plural press members 63b are provided around the outer periphery of a rotor 63 whose rotational shaft 63a is secured to the driving shaft of a DC motor 62, and a soft water feeding tube 64 are circumferentially arranged on the outer periphery of the rotor 63. The press members 63b of the rotor 63 rotates while crushing the water feeding tube 64, and pushes out liquid, thereby carrying out water feeding. To the motor 62 are connected a DC power source 65 and a variable resistor (volume) 60 for controlling the power (current) of the power source 65. The variable resistor 60 is designed to be operable by the flow rate adjusting finger grip 60F.

According to the variable resistor 60 having the flow rate adjusting finger grip 60F, the rotational number of the motor 62 is controlled by controlling the power amount (current amount) supplied from the DC power source 65, whereby the water feeding flow rate can be adjusted/controlled steplessly and continuously. As shown in Fig. 9, the arcuate flow rate graduating index is provided around the flow rate adjusting finger grip 60F, and for example, the flow rate from the minimum flow rate (min) of 90ml/sec till the maximum flow rate (max) of 200ml/sec can be adjusted/set.

Fig. 12 shows the construction of the pressure relief valve which can be utilized in place of the orifice 55. As shown in Fig. 5, the pressure relief valve 67 is disposed at the connection portion of the return pipe 58 on the side surface of the output port pipe 47. The pressure relief valve 67 has an opening/closing valve disc 67a and an urging member (spring or the like) 67b for moving the valve disc 67a forwardly and reversely, and the urging force of the urging member 67b is set so that the valve disc 67a is opened by the maximum water feeding pressure which is set to 29.4k Pascal or less. In this case, when the maximum water feeding pressure becomes abnormal, the liquid is made to flow out to the outside by opening the valve disc 67a, whereby the in-pipe pressure can be kept to be less than the maximum water feeding pressure. An elastic member formed of synthetic resin may be used for the urging member 67 and the elastic member may be formed integrally with the valve disc 67a.

### [example 3 not forming part of the claimed invention]

The overall construction of the water feeding device for an endoscope according to the example 3 is same as in the example 1 (See Fig. 1)

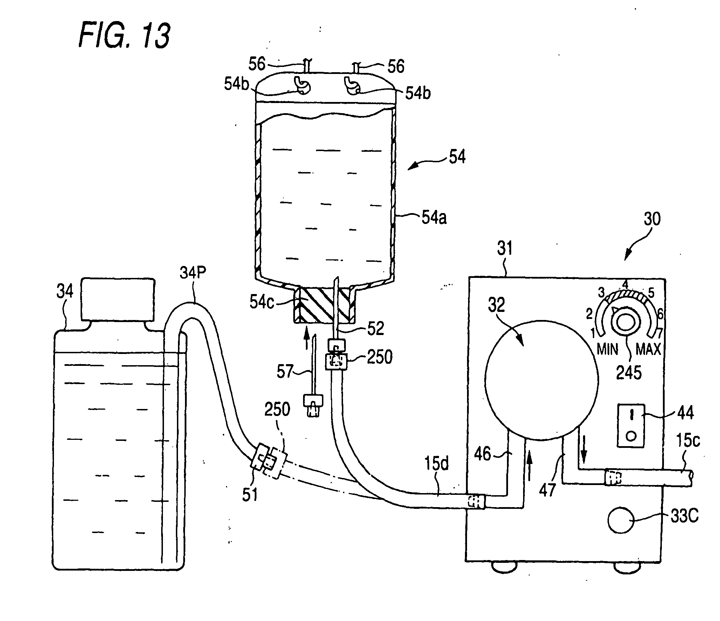

Fig. 13 shows the detailed constructions of the water feeding pump portion 30, the liquid bag and the tank 34. As shown in Fig. 1, the main body 31 of the water feeding pump portion (device) 30 is equipped with a power supply switch 44, a connection portion 33C of a foot switch 33, a flow rate adjusting finger grip (variable resistor) 245, etc. The connection pipe 15d at the tank side is connected to the input port pipe 46 of the pump 32, and the connection pipe 15c at the endoscope side is connected to the output port pipe 47 of the pump 32. A Luer connection type (male and female screw fixing type) female connector 50 is secured to the tip of the connection pipe 15d, and a male screw portion of the injection needle 52 and the female connector 50 screw together.

A liquid bag 54 is provided as a liquid container. The liquid bag 54 has the same construction as a fluid infusion (blood infusion) bag, and it comprises a soft synthetic resin main body bag 54a for stocking liquid such as physiological saline, drug solution or the like therein, a fitting hole 54b that is disposed at the upper portion of the main body bag 54a and fitted to a suspending hook 56 disposed in a dedicated stand, a system rack of the endoscope or the like, and a rubber liquid take-out portion 54c disposed at the lower portion of the main body bag 54a.

In the tank 34, the liquid take-out pipe 34P is disposed so as not to extend from the cap, but to extend from the upper portion of the side surface along the tank side surface to the bottom portion, and the Luer lock connection type male connector 51 to be connected to the female connector 50 is provided to the tip of the take-out pipe 34P.

According to the construction described above, the fitting hole 54b is fitted to the suspending hook 56 of Fig. 13 to suspend the liquid bag 54, and the injection needle 52 connected to the female connector 250 of the connection pipe 15d is stung into the rubber liquid take-out portion 54c of the liquid bag 54, whereby liquid such as physiological saline or the like in the liquid bag 54 can be supplied into the endoscope 10 side by the pump 32. At this time, a syringe (injection syringe) in which drug solution is filled is connected to another injection needle 57 shown in the figure, and the injection needle 57 is stung into the rubber liquid take-out portion 54c, whereby drug solution such as antifoaming agent or the like is properly added to the liquid and supplied from the water feeding pump portion 30.

The water jet pipe 15a of Fig. 1 and the second water feeding pipe 18 inserted from the forceps port 20 through the treatment instrument channel 16a can be switched to each other by the change-over valve 36, and target liquid can be fed into the object under observation through the water feeding pipe selected in accordance with the condition.

According to the liquid bag 54, the liquid container can be set as a disposal (only-once usable) liquid container, and also no air exists in the container, so that all the liquid can be taken out from the injection needle 52 with no residual.

Furthermore, after the injection needle 52 is detached from the Luer lock connection type female connector 50, the male connector 51 of the take-out pipe 34P of the tank 34 may be connected to the female connector 50 to use the liquid in the tank 34 as shown in Fig. 13. That is, the liquid bag 54 and the tank 34 having the take-out pipe 34P can be selected in accordance with the situation.

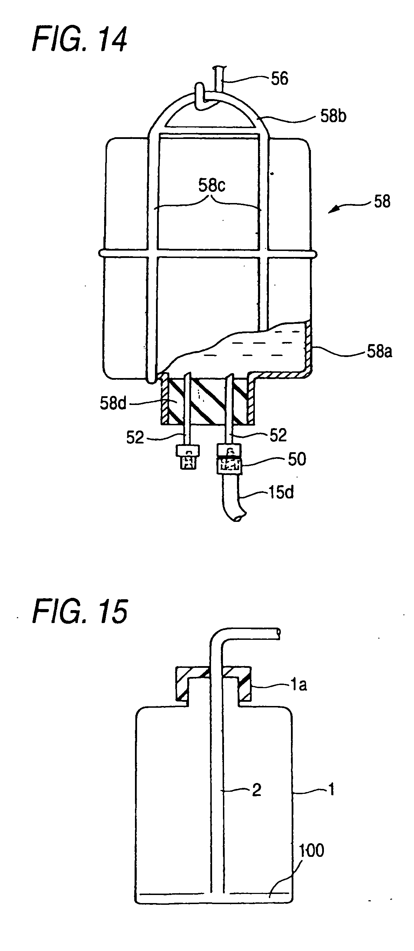

Fig. 14 shows another example of the liquid container. In this example, a soft bag is not used, but a hard container is used. That is, the liquid container 58 comprises a main body container 58a formed of hard synthetic resin or glass container or the like, a mesh-type supporter 58b that has a fitting portion 58b at the upper portion thereof and wound around the outer periphery of the main body container 58a so as to hold the main body container 58a, and a rubber liquid take-out portion 58d.

In the case of the above-described liquid container 58, the liquid container 58 is also suspended by fitting the fitting portion 58b to the suspending hook 56, the injection needle 52 connected to the connection pipe 15d is stung into the rubber liquid take-out portion 58d of the liquid container 58, and also another injection needle 52 for securing an air supply passage is stung into the rubber liquid take-out portion 58d, whereby the liquid such as physiological saline or the like in the liquid container 58 can be supplied to the endoscope 10 side by the pump 32.

## Claims

1. A water feeding device for an endoscope (10), comprising:
a water feeding pump portion (30) that supplies liquid in a storage portion (34) by a pump (32,32A,32B);
a water feeding pipe line (15a,15b,15c,18) that is disposed so as to pass from the water feeding pump portion (30) through the endoscope (10) and supplies liquid to an object under observation; and
one of an orifice (55) and an automatic opening/closing type pressure relief valve (67), that limits the maximum water feeding pressure and is provided to the water feeding pipe line (15b,15c,18) disposed between the water feeding pump portion (30) and the endoscope (10)
**characterised by** a pipe line (58) that returns liquid flowing out from said one of the orifice (55) and the automatic opening/closing type pressure relief valve (67) to the storage portion (34), the pipe line (58) being provided between: one of the orifice (55) and the pressure relief valve (67), and the storage portion (34).

2. The water feeding device for the endoscope (10) according to claim 1,
wherein at least one of a first water feeding pipe line (15b) connected to the water feeding pipe line (15a) in the endoscope (10) and a second water feeding pipe line (18) to be introduced through a treatment instrument insertion channel (17a) of the endoscope (10) is connected to the water feeding pump portion (30) and
said one of the orifice (55) and the automatic opening/closing type pressure relief valve (67) is provided to an output port pipe (47) connected to said at least one of the first (15b) and second (18) Water feeding pipe.

3. The water feeding device for an endoscope (10) according to claim 1,
wherein the storage portion (34) comprises a liquid container (54), the liquid container (54) comprising: a fitting member (54b) that is fitted to and suspended by a suspending member (56), a main body (54a) that stocks liquid; and a rubber liquid take-out portion (54c) that is disposed at a lower portion of the main body (54a) and through which an inj ection needle (52) penetrates
wherein the water feeding device further comprises a connection pipe line (15d) that is connected to the water feeding pump portion (30) and to which the injection needle (52) is secured, and
wherein liquid is fed from the liquid container (54) to the water feeding pump portion (30) under the state that the liquid container (54) is suspended by the suspending member (56) and the injection needle (52) is stung.

4. The water feeding device for the endoscope (10) according to claim 3.
wherein the connection pipe line (15d) to which the injection needle (52) is secured comprises a connector (250) that selectively connecting the injection needle (52) and a take-out pipe line (34P) equipped to a tank (34)

## Patentansprüche

1. Wasserzuführungsvorrichtung für ein Endoskop (10) mit:
einem Wasserzuführungspumpenteil (30), der Flüssigkeit in einem Aufbewahrungsteil (34) durch eine Pumpe (32, 32A, 32B) liefert,
einer Wasserzuführungsrohrleitung (1 5a, 15b, 1 5c, 18), die so angeordnet ist, dass sie von dem Wasserzuführungspumpenteil (30) durch das Endoskop (10) führt und Flüssigkeit zu einem unter Beobachtung stehenden Objekt liefert und
eines von einer Öffnung (55) und einem Druckentlastungsventil (67) vom automatisch öffnenden/schließenden Typ, das einen maximalen Wasserzuführungsdruck begrenzt und für die Wasserzuführungsrohrleitung (1 5b, 15c, 1 8) vorgesehen ist und zwischen dem Wasserzuführungspumpenteil (30) und dem Endoskop (10) angeordnet ist,
**gekennzeichnet durch** eine Rohrleitung (58), die Flüssigkeit, die aus dem einen der Öffnung (55) und des Druckentlastungsventils (67) vom automatisch öffnenden/schließenden Typ ausströmt, zum Aufbewahrungsteil (34) zurückführt, wobei die Rohrleitung zwischen: einem der Öffnung (55) und des Druckentlastungsventils (67) und dem Aufbewahrungsteil (34) vorgesehen ist.

2. Wasserzuführungsvorrichtung für ein Endoskop (10) nach Anspruch 1,
wobei zumindest eine einer ersten Wasserzuführungsrohrleitung (1 5b), welche mit der Wasserzuführungsrohrleitung (15a) in dem Endoskop (10) verbunden ist, und einer zweiten Wasserzuführungsrohrleitung (18), welche durch einen Behandlungsinstrurnent-Einführungskanal (17a) des Endoskops (10) eingeführt wird, mit dem Wasserzuführungspumpenteil (30) verbunden ist und
wobei das eine der Öffnung (55) und des Druckentlastungsventils (67) vom automatisch öffnenden/schließenden Typ an einem Ausgangsöffnungsrohr (47) vorgesehen ist, welches mit mindestens einen des ersten (15b) und des zweiten (18) Wasserzuführungsrohrs verbunden ist.

3. Wasserzuführungsvorrichtung für ein Endoskop (10) nach Anspruch 1,
wobei der Aufbewahrungsteil (34) einen Flüssigkeitsbehälter (54) umfasst, wobei der Flüssigkeitsbehälter (54) umfasst: ein Installationselement (54b), das an einem Aufhängungselement (56) installiert und aufgehängt wird; einen Hauptkörper (54a), der Flüssigkeit lagert; und einen Gummi-Flüssigkeitsentnahmeteil (54c), der an einem unteren Teil des Hauptkörpers (54a) angeordnet ist und durch den eine Injektionsnadel (52) eindringt,
wobei die Wasserzuführungsvorrichtung ferner eine Verbindungsrohrleitung (15d) umfasst, die mit dem Wasserzuführungspumpenteil (30) verbunden ist und an der die Injektionsnadel (52) befestigt ist, und
wobei Flüssigkeit vom Flüssigkeitsbehälter (54) zum Wasserzuführungspumpenteil (30) in dem Zustand zugeführt wird, in dem der Flüssigkeitsbehälter (54) durch das Aufhängungselement (56) aufgehängt ist und die Injektionsnadel (52) eingestochen ist.

4. Wasserzuführungsvorrichtung für ein Endoskop (10) nach Anspruch 3,
wobei die Verbindungsrohrleitung (1 5d), an der die Injektionsnadel (52) befestigt ist, ein Verbindungselement (250) umfasst, das selektiv die Injektionsnadel (52) und eine Entnahmerohrleitung (34P), mit der ein Tank (34) ausgestattet ist, verbindet.

## Revendications

1. Dispositif d'alimentation en eau pour un endoscope (10), comportant :
une partie de pompe d'alimentation en eau (30) qui délivre du liquide dans une partie de stockage (34) grâce à une pompe (32, 32A, 32B) ;
une conduite d'alimentation en eau (15a, 15b, 15c, 18) qui est disposée afin de passer depuis la partie de pompe d'alimentation en eau (30) à travers l'endoscope (10) et délivre du liquide à un objet sous observation ; et
un orifice (55) ou un clapet de libération de pression du type à ouverture/fermeture automatique (67) qui limite la pression d'alimentation en eau maximum et est prévu sur la conduite d'alimentation en eau (15b, 15c, 18) disposée entre la partie de pompe d'alimentation en eau (30) et l'endoscope (10),
**caractérisé par**
une conduite (58) qui renvoie du liquide s'écoulant hors dudit orifice (55) ou dudit clapet de libération de pression du type à ouverture/fermeture automatique (67) dans la partie de stockage (34), la conduite (58) étant prévue entre : l'orifice (55) ou le clapet de libération de pression (67), et la partie de stockage (34).

2. Dispositif d'alimentation en eau pour l'endoscope (10) selon la revendication 1,
dans lequel au moins une première conduite d'alimentation en eau (15b) reliée à la conduite d'alimentation en eau (15a) dans l'endoscope (10) ou une deuxième conduite d'alimentation en eau (18) devant être introduite dans un canal d'insertion d'instrument de traitement (17) de l'endoscope (10) est reliée à la partie de pompe d'alimentation en eau (30), et
ledit orifice (55) ou ledit clapet de libération de pression du type à ouverture/fermeture automatique (67) est prévu dans un tube d'orifice de sortie (47) relié à ladite au moins une première (15b) ou deuxième (18) conduite d'alimentation en eau.

3. Dispositif d'alimentation en eau pour un endoscope (10) selon la revendication 1,
dans lequel la partie de stockage (34) comporte un réservoir de liquide (54), le réservoir de liquide (54) comportant : un élément de montage (54b) qui est monté sur et suspendu par un élément de suspension (56), un corps principal (54a) qui stocke du liquide ; et une partie de sortie de liquide en caoutchouc (54c) qui est disposée au niveau d'une partie inférieure du corps principal (54a) et à travers laquelle pénètre une aiguille d'injection (52),
dans lequel le dispositif d'alimentation en eau comporte en outre une conduite de raccordement (15d) qui est reliée à la partie de pompe d'alimentation en eau (30) et sur laquelle l'aiguille d'injection (52) est fixée, et
dans lequel le liquide est délivré depuis le réservoir de liquide (54) à la partie de pompe d'alimentation en eau (30) dans l'état où le réservoir de liquide (54) est suspendu par l'élément de suspension (56) et l'aiguille d'injection (52) est plantée.

4. Dispositif d'alimentation en eau pour l'endoscope (10) selon la revendication 3,
dans lequel la conduite de raccordement (15d) sur laquelle l'aiguille d'injection (52) est fixée comporte un raccord (250) qui raccorde de manière sélective l'aiguille d'injection (52) et une conduite de sortie (348) prévue sur un réservoir (34).
